# EUROPEAN PATENT APPLICATION

(11) **EP 4 085 855 A1**
(43) Date of publication of application: **09.11.2022**
(21) Application number: 21305576.7
(22) Date of filing: 04.05.2021
(51) Int. Cl.: A61B 17/42

(54) **HEMOSTATIC DEVICE**

(71) Applicant: Hemosquid, 38400 Saint Martin d'Heres (FR)
(72) Inventor: OTT, Franck, 38440 SAINT JEAN DE BOURNAY (FR); BARRIER, Pascal, 74000 ANNECY (FR); LAVALLEE, Stéphane, 38410 SAINT MARTIN D'URIAGE (FR); FALCO, Eric, 38000 GRENOBLE (FR)
(74) Representative: Regimbeau

(57) **Abstract**

The disclosure relates to a hemostatic device (1) comprising at least a first chamber (100) configured to be fluidically connected to a fluid source and a second chamber (200) configured to be fluidically connected to a vacuum source,
wherein the hemostatic device comprises a first membrane (201) fluidically isolating the second chamber (200) from the first chamber (100) and a second membrane (202) configured to be placed so as to face, at least partially, a bleeding area of a natural body cavity, the second membrane (202) comprising a plurality of through holes (203) leading into the second chamber (200) and configured to induce a negative pressure in the natural body cavity when a negative pressure is applied in the second chamber (200) by the vacuum source, the induced negative pressure being configured so that the walls of the natural cavity are attracted to the second membrane (202).

## Description

### TECHNICAL FIELD

The present disclosure relates to a hemostatic device, in particular intended to stop or reduce hemorrhages in a natural body cavity such as a uterus.

### TECHNICAL BACKGROUND

After childbirth, the mother's uterus is generally subjected to contractions that allow to stop the bleeding caused by the detachment of the placenta.

However, in some cases, the uterus may suffer from atony, so that such contractions do not occur or are not sufficient to stop bleeding, thereby generating a risk of maternal hemorrhage.

Various hemostatic devices have been developed to promote the contraction of the uterus and help stopping or reducing the hemorrhage.

Document EP 3 248 624 teaches a hemostatic device for treating postpartum hemorrhage, comprising a flat flexible plate presenting a pair of opposite faces configured to be placed in contact with a wall of the uterus and an empty internal volume in fluidic connection with a vacuum pump. Each face comprises a plurality of holes such that when a negative pressure is created in the internal volume by the pump, the walls of the uterus are attracted by the respective face of the plate and held together, which mechanically shrinks the uterus, thereby compacting the muscles forming the wall of the uterus and constricting the blood vessels to stop bleeding. However, the size of the uterus may vary from one patient to another one. It may thus be necessary to provide different formats of the hemostatic device to allow the practitioner to select the one fitting best the patient's uterus. In addition, the size of the uterus also decreases as the uterus contracts. Thus, once the uterus has attained the size of the flexible plate, the hemostatic device may hinder further contraction of the uterus.

Document US 2005/0015047 teaches a hemostatic device for treating postpartum hemorrhage, comprising an inner inflatable balloon in fluidic connection with a gas source and an outer inflatable balloon extending about the inner balloon. To introduce the hemostatic device into the uterus, the balloons are in a non-inflated state. Once the hemostatic device is in the uterus, gas from the gas source is supplied to the inner balloon to inflate it to conform to the wall of the uterus. The outer balloon is intended to confine the gas, for example in case of leakage of the inner balloon.

Document US 2017/0035949 teaches a hemostatic device for treating postpartum hemorrhage, comprising a foam body comprising open cells in fluidic connection with a vacuum pump.

Document WO 2020/123525 teaches a hemostatic device for treating postpartum hemorrhage, comprising a flexible portion configured to be inserted into the uterus, a seal configured to close the opening of the uterus, and a vacuum pump in fluidic connection with holes provided in the flexible portion. When the uterus is sealed, the pump creates a negative pressure within the uterus via the holes in order to facilitate contractile movement of the uterine wall and vessel constriction. In this device, various means may be provided to protect the holes from occlusion by tissues.

### SUMMARY OF THE DISCLOSURE

There remains a need for a hemostatic device that can be easily inserted into a natural body cavity such as the uterus and conform to the shape of the cavity, while providing an efficient stimulation of the muscles forming the wall of the cavity and constriction of the blood vessels, without hindering further contraction of the uterus over time.

Some embodiments relate to a hemostatic device comprising at least a first chamber configured to be fluidically connected to a fluid source and a second chamber configured to be fluidically connected to a vacuum source,
wherein the hemostatic device comprises a first membrane fluidically isolating the second chamber from the first chamber and a second membrane configured to be placed so as to face, at least partially, a bleeding area of a natural body cavity, the second membrane comprising a plurality of through holes leading into the second chamber and configured to induce a negative pressure in the natural body cavity when a negative pressure is applied in the second chamber by the vacuum source, the induced negative pressure being configured so that the walls of the natural cavity are attracted to the second membrane.

The first chamber is expandable by a fluid pressure from a retracted state allowing insertion of the hemostatic device into a natural body cavity (for example, a uterus) to an expanded state fitting said internal cavity.

In some embodiments, the second chamber extends at least partially around the first chamber.

In other embodiments, the first chamber extends at least partially around the second chamber, the first chamber being enclosed between the second membrane and the first membrane, the first membrane comprising through holes in fluidic communication with the through holes of the second membrane by tunnels passing through the first chamber.

The second chamber may comprise an internal structure mechanically connecting the first and second membranes to prevent collapsing of the second membrane when a negative pressure is applied in the second chamber.

Said internal structure may comprise at least one pillar or wall extending between the first and second membranes so as to define, in the second chamber, at least two cavities in fluidic communication with each other.

Preferably, at least one of the plurality of through holes is arranged in the second membrane of each respective cavity.

Advantageously, the through holes may be arranged in the second membrane according to a regular pattern.

In some embodiments, the first and second membranes present a corrugated shape such that the first and second membranes are foldable in at least one direction when a compressive force is applied to the hemostatic device in said at least one direction.

The first and second membranes may also or alternatively present a corrugated shape such that the first and second membranes are expandable when a positive pressure is applied in the first chamber.

In some embodiments, the first chamber may comprise an internal lattice or foam selectively collapsible or expandable between a retracted configuration and an expanded configuration.

The hemostatic device may further comprise a base coupled to the first and second chambers, wherein the base comprises
at least one first connecting tube extending between the first chamber and a face of the base, and
at least one second connecting tube extending between the second chamber and a face of the base.

The hemostatic device may further comprise an insertion rod mechanically connected to the base, the insertion rod comprising a first channel in fluidic communication with the first connecting tube and connected to the fluid source and a second channel in fluidic communication with the second connecting tube and connected to the vacuum source.

The insertion rod may advantageously be connected to the base by a flexible member.

Another object of the present disclosure is a process for manufacturing a hemostatic device as described above, comprising forming the first and second membranes by a layer-by-layer process in which each layer is made of a biocompatible polymer.

Said hemostatic device may be used according to the following method:
- provision of the hemostatic device in a retracted state;
- insertion of the hemostatic device in said retracted state into a natural cavity;
- applying a fluid pressure within the first chamber;
- applying a negative pressure within the second chamber so as to attract the walls of the natural cavity;
- maintaining said negative pressure;
- gradually retracting the hemostatic device by decreasing the fluid pressure within the first chamber until the hemostatic device is again in the retracted state;
- maintaining the negative pressure for a minimal treatment time;
- stopping the negative pressure;
- removing the hemostatic device in the retracted state from the natural cavity.

### BRIEF DESCRIPTION OF THE FIGURES

Further features and advantages will be described in the following description, based on the appended drawings, in which:
- FIG. 1 is a sectional view of a hemostatic device according to a first embodiment;
- FIG. 2 is a sectional view of a hemostatic device according to a second embodiment;
- FIG. 3 schematically illustrates the operation of the hemostatic device of FIG. 2 when placed in a natural body cavity;
- FIG. 4 schematically illustrates successive steps of operation of the hemostatic device;
- FIG. 5 is a perspective overall view of the hemostatic device;
- FIG. 6A is a side view of a hemostatic device according to a third embodiment;
- FIG. 6B is a sectional view of the hemostatic device of FIG. 6A;
- FIG. 6C is an enlarged view of a portion of FIG. 6B;
- FIG. 7 is a view of the hemostatic device with a lattice inside the first chamber;
- FIG. 8 schematically illustrates a fourth embodiment of the hemostatic device, comprising a dual-membrane separating the second chamber from the organ internal cavity, the first chamber being encapsulated in said dual-membrane.

For sake of legibility of the drawings, some components of the hemostatic device may have been omitted. In addition, the drawings are not necessarily drawn to scale.

In the drawings, identical reference signs designate elements that are identical to each other or that fulfil the same function. Thus, when one element has been described in detail with reference to one figure, it may not be described in detail again with reference to another figure.

### DETAILED DESCRIPTION OF EMBODIMENTS

The hemostatic device comprises at least two chambers.

A first chamber is configured to be fluidically connected to a fluid source and a second chamber is configured to be fluidically connected to a vacuum source.

The first and second chambers are fluidically isolated from each other by a first membrane. Said first membrane prevents any circulation of fluid between the first chamber and the second chamber. As a result, the first chamber may be inflated by injection of fluid from the fluid source while the second chamber may be put under a negative pressure by the vacuum source.

The hemostatic device comprises a second membrane configured to be placed so as to face, at least partially, a bleeding area of a natural body cavity. The second membrane comprises a plurality of through holes leading into the second chamber and configured to induce a negative pressure in the natural body cavity when a negative pressure is applied in the second chamber by the vacuum source.

The following description is directed to the treatment of postpartum hemorrhage, the natural cavity being a uterus. However, the hemostatic device may also be used to treat hemorrhage of other natural cavities, such as stomach, bowel, oesophagus, tumor cavity... (non-limitative list). The shape and size of the hemostatic may be vary depending on intended natural cavity, in order to best fit the shape and size of said natural cavity.

Thanks to the first chamber, the hemostatic device is deformable between a retracted position, in which the fluid pressure in the first chamber is relatively low, and an expanded position, in which the fluid pressure in the first chamber is relatively high. The retracted position is configured to allow easy insertion of the hemostatic device into the uterus, while the expanded position is configured to allow conforming the size and shape of the hemostatic device to the uterus. In addition, the transition from the expanded position to the retracted position as the hemostatic device is within the uterus allows conforming to the size and shape of the natural cavity as the uterus retracts over time during the medical procedure. Otherwise said, the hemostatic device does not oppose the natural retraction of the uterus over time. At the end of the medical procedure, the hemostatic device may be brought back to the initial retracted position and may thus be easily removed from the uterus.

Thanks to the holes provided in the second membrane to lead into the second chamber and thus fluidically connect the second chamber and the internal cavity of the uterus, a negative pressure is induced within the uterus, said induced negative pressure being configured so that the walls of the uterus are attracted to the second membrane and maintained firmly against the second membrane as long as the negative pressure is maintained by the vacuum source. The engagement of the second membrane with the walls of the natural cavity has the effect of initiating hemostasis thanks to the tight contact between the walls and the second membrane, which exerts a compression onto the bleeding vessels, and further of stimulating a muscular contraction of the uterus and the constriction of the blood vessels in the uterine tissues. Thus, even if a small quantity of blood may be aspirated into the second chamber via the through holes at the beginning of the application of the negative pressure, such an aspiration of blood quickly stops thanks to a prompt reduction of the bleeding.

The first and second membranes advantageously present a certain elasticity, in order to allow the hemostatic device to be folded, flattened or, more generally, retracted, to facilitate its insertion into the natural body cavity, or its extraction from the natural cavity. On the other hand, the first membrane allows inflating the hemostatic device to its expanded position.

Preferably, the first and second membranes, and more generally all the components of the hemostatic device that are intended to be placed inside the patient's body, are made of a biocompatible material.

For example, the first and second membranes may be formed of a biocompatible elastomeric material, such as Silicone / Silicone urethane / Thermoplastic Elastomer (TPE) family (including in particular Thermoplastic Polyurethane (TPU), etc) / Polyurethane elastomer (EPU), etc.

The first and second membranes may have a thickness comprised between 0.1 and 5 mm. Advantageously, the second membrane may have a thickness sufficiently high to limit collapsing of said membrane when a negative pressure is applied in the second chamber. Said thickness may depend on the stiffness of the material of the second membrane. The thickness of the first membrane may be equal to or different from the thickness of the second membrane.

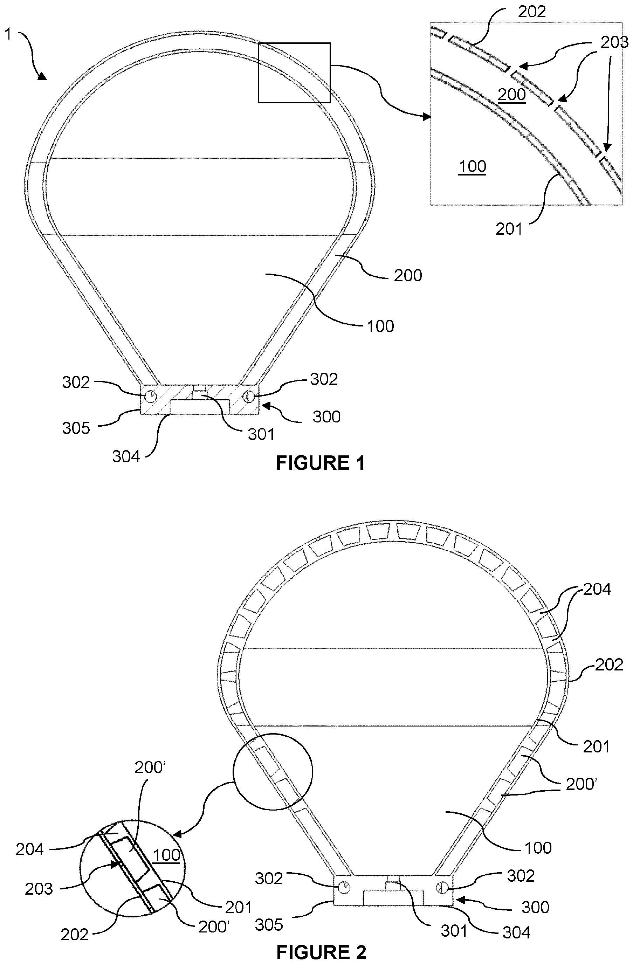

In the following description, based on FIGS. 1-7, it is assumed that the second chamber extends at least partially around the first chamber. In such case, the first chamber may be enclosed in the first membrane and the second chamber may be enclosed between the first and second membranes.

However, other arrangements of the first and second chambers may be implemented. Besides, the number of chambers may be varied, provided that the device comprises at least one chamber configured to be connected to the vacuum source and at least one chamber configured to be connected to the fluid source.

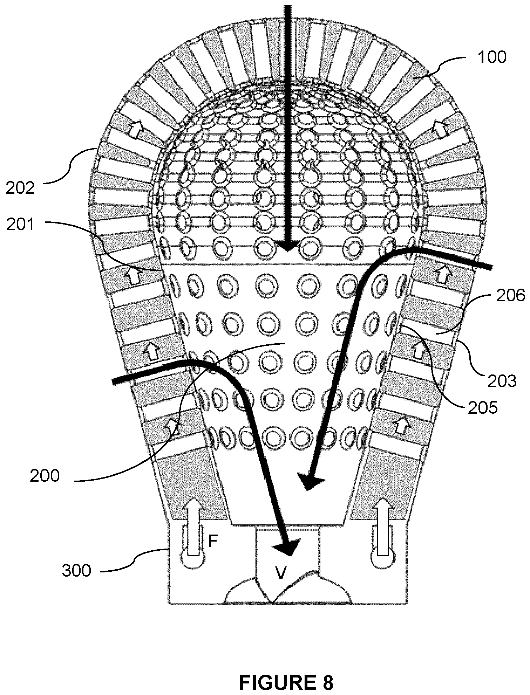

For example, in some embodiments, as shown in FIG. 8, the first chamber 100 may extend at least partially around the second chamber 200. In this case, the first chamber 100 is enclosed between the second membrane 202 and the first membrane 201. The first chamber 100 is configured to expand with inner fluid pressure brought by the fluid source (arrow F). The second chamber 200 is connected to the vacuum source for application of a negative pressure (arrow V). The first membrane comprises through holes 205 that are connected to the through holes 203 of the second membrane 202 by tunnels 206 that extend through the first chamber 100. Thus, although the second chamber is located inside the hemostatic device and not in direct contact with the second membrane which is to be applied onto the walls of the natural cavity, the tunnels 206 extending through the first chamber allow fluidically connecting the second chamber and the natural cavity to apply the negative pressure into said cavity.

In some embodiments, the device may include two or more first chambers, each of said first chambers being configured to be connected to the fluid source. Said first chambers may be connected independently to the fluid source, for example via respective fluid channels extending from the fluid source and each first chamber. Otherwise, the first chambers may be in fluidic communication with each other, one of said first chambers being connected to the fluid source.

In some embodiments, the device may include two or more second chambers, each of said second chambers being configured to be connected to the vacuum source. Said second chambers may be connected independently to the vacuum source, for example via respective fluid channels extending from the vacuum source and each second chamber. Otherwise, the second chambers may be in fluidic communication with each other, one of said second chambers being connected to the vacuum source. Similarly, said second chambers may be connected independently to the organ internal cavity.

Said embodiments may be combined by the skilled person, depending on the intended use of the hemostatic device, the first and second chambers being arranged relative to each other in order to provide an optimal hemostasis.

FIG. 1 illustrates an embodiment of the hemostatic device 1 comprising a first, inner chamber 100 configured to be connected to a fluid source (not shown) and a second, outer chamber 200, configured to be connected to a vacuum source (not shown) and arranged around the first chamber 100.

The second, outer chamber 200 comprises a first, inner membrane 201 fluidically isolating the second chamber 200 from the first chamber 100 and a second, outer membrane 202 configured to be placed so as to face, at least partially, a bleeding area of a natural body cavity (not illustrated).

As better seen in the enlarged partial view, the second, outer membrane 202 comprises a plurality of through holes 203.

The size, number and arrangement of the through holes over the surface of the second membrane are chosen to induce a negative pressure in the natural body cavity when a negative pressure is applied in the second chamber by the vacuum source, the induced negative pressure being configured so that the walls of the natural cavity are attracted to the second membrane.

The through holes advantageously all have the same shape and size. For example, the through holes all have a circular shape, with a diameter comprised between 0.2 and 5 mm.

Preferably, the through holes 203 are arranged according to a regular pattern. By "regular pattern" is meant in the present text that the density of through holes by surface unit is substantially constant over the surface of the second membrane. For example, the density of through holes is comprised between 0.1 and 10 holes/cm² and the distance between two holes does not vary by more than 100% over the surface of the second membrane.

Thanks to said regular pattern of the through holes, the negative pressure applied by the hemostatic device in the natural cavity is substantially uniform.

In the embodiment of FIG. 1, both the first and second chambers are empty from any solid material. Otherwise said, said chambers only comprise air or another gas, or even (in the case of the first chamber) a liquid.

However, in other embodiments, the first and/or the second chamber may comprise an internal structure.

FIG. 2 illustrates an embodiment of the hemostatic device in which the second chamber 200 comprises an internal structure 204 mechanically connecting the first and second membranes 201, 202 to prevent collapsing of the second membrane 202 when a negative pressure is applied in the second chamber 200.

The rest of the structure of the hemostatic device is similar to the structure of the embodiment of FIG. 1.

The internal structure 204 may comprise at least one pillar and/or at least one wall extending between the first and the second membrane, or a combination of such pillars and walls. In this way, the internal structure 204 prevents the second membrane 202 from collapsing onto the first membrane 201 when a negative pressure is applied in the second chamber 200 by the vacuum source. Indeed, thanks to the internal structure, there always remains a volume between the first and second membranes in which the negative pressure applied by the vacuum source can be propagated.

The internal structure may be fixed to both membranes, or to only one of the membranes. In the latter case, the other membrane may come into contact with the internal structure when a negative pressure is applied in the second chamber, but the internal structure prevents the membranes from coming in contact with each other. For example, the internal structure may comprise conic pillars (see FIG. 2) or picots preventing the collapse of the second chamber.

The internal structure may divide the second chamber 200 into a plurality of cavities 200'. In order to allow the negative pressure to be applied throughout the whole second chamber, the internal structure may be arranged so that said cavities 200' are in fluidic communication with each other. In this way, the negative pressure applied by the vacuum source may be propagated to all cavities 200'. Preferably, each cavity 200' comprises at least one through hole 203.

The internal structure may be made of an elastomeric material, which may be the same material as the first and second membranes. Preferably, the internal structure may be integrally formed with at least one of the first and second membranes.

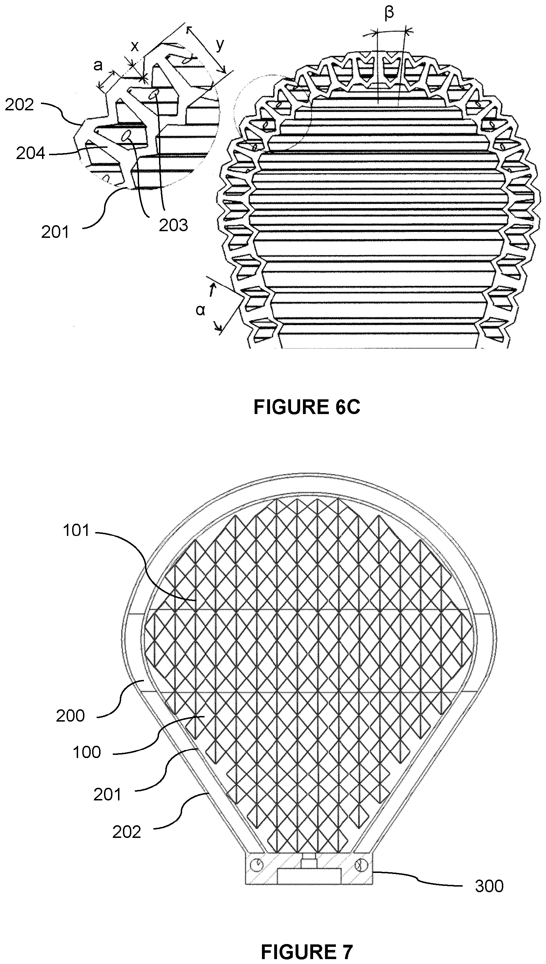

FIG. 7 illustrates an embodiment of the hemostatic device in which the first chamber 100 comprises an internal structure 101.

The rest of the structure of the hemostatic device is similar to the structure of the embodiment of FIG. 1.

The internal structure may be in the form of a lattice comprising a plurality of facets or segments articulated together so as to allow retraction or expansion of the first chamber. Alternatively, the internal structure may be formed of a foam which is elastically compressible to allow retraction or expansion of the first chamber.

In combination with the fluid pressure provided by the fluid source, the internal structure allows reinforcing the first chamber in order to obtain the desired shape in the expanded state.

The internal structure 101 may be fixed to the first membrane 201 (and/or to the second membrane in an alternative embodiment such as illustrated in FIG. 8).

The internal structure may be made of an elastomeric material, which may be the same material as the first and/or second membranes. Preferably, the internal structure may be integrally formed with the first membrane and/or second membrane.

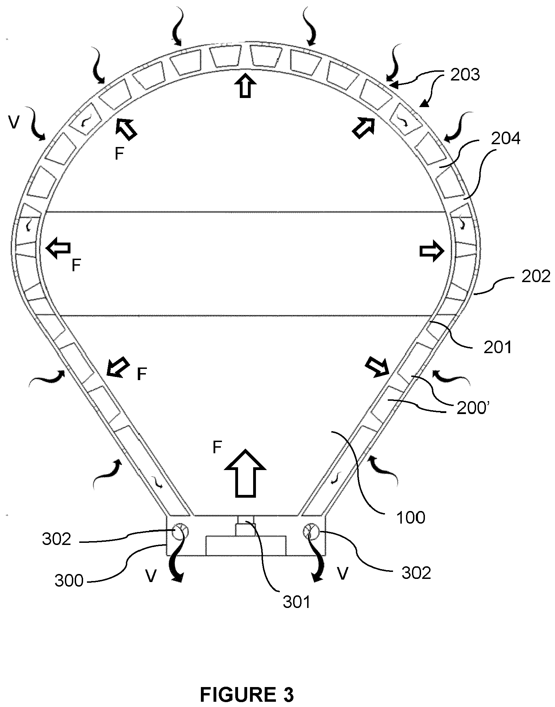

The effect of the hemostatic device will be described with reference to FIG. 3.

The hemostatic device of FIG. 3 is identical to FIG. 2, but the direction of the fluid pressure applied in the first chamber 100 is represented by straight arrows F and the negative pressure applied in the second chamber 200 to aspirate the walls of the natural cavity is represented by curved arrows V.

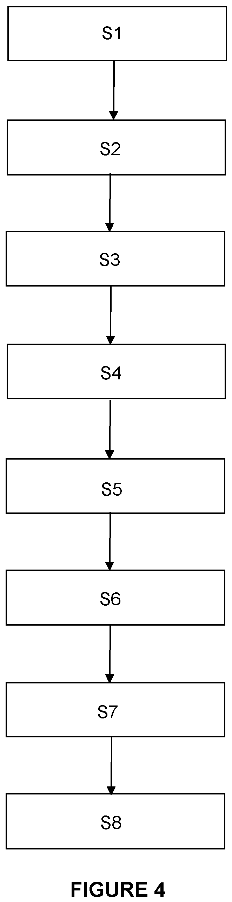

A complete workflow of a treatment using the hemostatic will be described with reference to FIG. 4.

In step S1, the hemostatic device is prepared by the medical staff. The preparation includes in particular unpacking the device from its package and connecting the fluidic accesses to the fluid source and the vacuum source.

In step S2, the hemostatic device is inserted into the natural cavity, for example the uterus.

In step S3, the hemostatic is pressurized by injecting fluid into the first chamber from the fluid source. As a result, the device adapts to the morphology of the natural cavity.

In step S4, the second chamber is evacuated by the vacuum source. As a result, the uterus walls are attracted by the second membrane.

Step S5 is a first stabilization step, in which the vacuum is maintained. Hemostasis is initiated by a dual action of the hemostatic device: (1) direct contact of the uterus wall to the device (promoting a so-called "contact hemostasis") and (2) compression of the wall and its internal structure by aspiration.

In step 6, the hemostatic device is gradually retracted by decreasing the fluid pressure within the first chamber. At the end of said step, the device reaches the minimum recommended size. During said step, the uterus size also decreases, and a negative pressure may be maintained by the vacuum source.

Step S7 is a second stabilization step, in which the reduction of the size of the uterus promotes the contraction of the muscles. These contractions compress the vessels and stop the bleeding permanently. During said step, a negative pressure may be maintained by the vacuum source.

In step S8, the negative pressure is stopped.

In step S9, the hemostatic device is removed from the natural cavity, and the medical procedure ends, after a minimal treatment time of 10 minutes for example, depending on the specific protocol.

To allow connecting the first chamber to the fluid source and the second chamber to the vacuum source, the hemostatic device advantageously comprises a base coupled to the first and second chambers, and comprising connecting tubes passing through the base.

FIGS. 1, 2 and 5 illustrate an embodiment of such a base 300. The base may have a substantially cylindrical shape with a peripheral cylindrical face and two opposite end faces. The first and second chambers extend from one of said faces. A first connecting tube 301 extends between the first chamber 201 and the opposite face 304 of the base. Two second connecting tubes 302 extend between the second chamber and the peripheral face 305 of the base.

In this embodiment, the arrangement of the first connecting tube in a central region of the base and the arrangement of the second connecting tubes in a peripheral region of the base are particularly advantageous in view of the position of the second, outer chamber around the first, inner chamber. However, the skilled person is of course able to select another shape of the base and another arrangement of the first and second connecting tubes depending on the specific arrangement of the first and second chambers. For example, the connecting tubes may extend from a same face of the base.

Although the base may be made of the same material as the first and second chambers, it is preferably more rigid in order to facilitate its mechanical connection to an insertion rod adapted to be manipulated by a practitioner to assist insertion and removal of the hemostatic device withing a natural cavity.

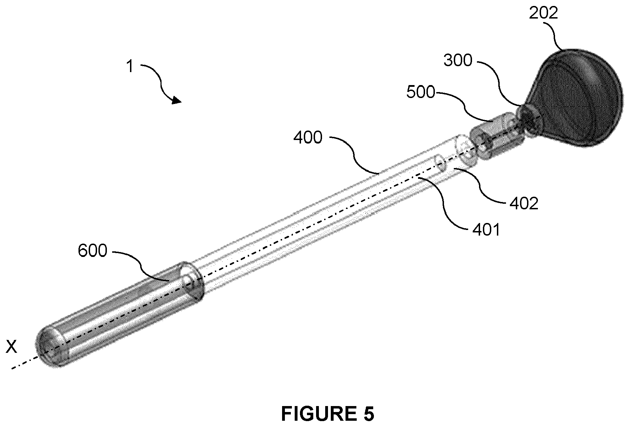

As shown in FIG. 5, the insertion rod 400 has a substantially cylindrical shape extending around a longitudinal axis X. The insertion rod is mechanically connected to the base 300, preferably by a flexible member 500 as will be explained below.

The insertion rod advantageously comprises a first channel 401 in fluidic communication with the first connecting tube 301 and connected to the fluid source and a second channel 402 in fluidic communication with the second connecting tube 302 and connected to the vacuum source. In the illustrated embodiment, the first and second channels are coaxial, the second channel being arranged around the first channel. However, the skilled person may select a different arrangement of the first and second channels, depending in particular of the position of the first and second connecting tubes of the base.

The insertion rod 400 may be connected to the base 300 by a flexible member 500. The flexible member may be formed of a biocompatible elastomeric material. The flexible member is configured to be able to deflect relative to the longitudinal axis X, in order to facilitate orientation of the hemostatic device during the insertion into the natural cavity. The insertion rod comprises fluidic channels adapted to fluidically connect the first channel of the insertion rod to the first connecting tube of the base and the second channel of the insertion rod to the second connecting tube of the base.

At the end of the insertion rod 400 opposite to the base 300, a syringe-like piston 600 may be arranged. Activation of the syringe-like piston 600 pushes the fluid of the first channel 401 into the first chamber 100 which expands.

In the embodiments shown in FIGS. 1 and 2, the first and second membranes have a substantially smooth shape. However, it may be advantageous to provide said membranes with a corrugated shape. Such a corrugated shape may be configured so that the first and second membranes are foldable in at least one direction when a compressive force is applied to the hemostatic device in said at least one direction. In addition or alternatively, the corrugated shape may be configured so that the first and second membranes are expandable when a positive pressure is applied in the first chamber. Moreover, such corrugated shape geometry can vary from the bottom to the top of the device.

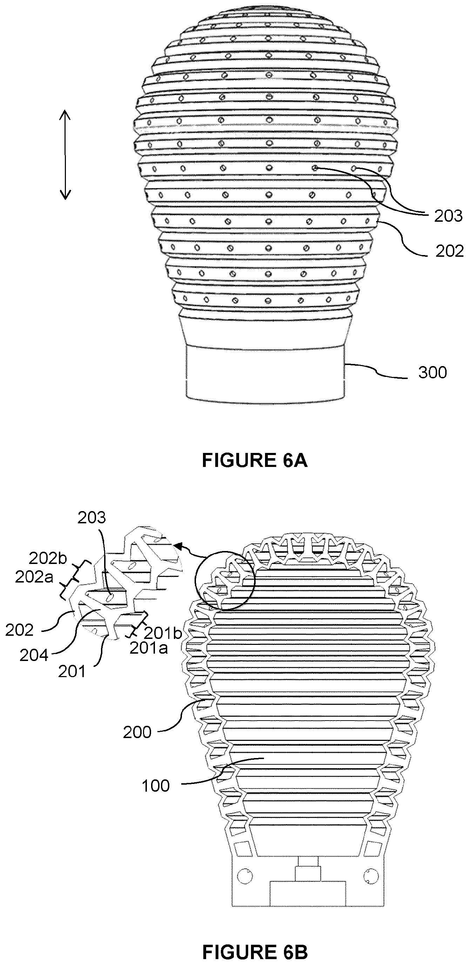

FIGS. 6A and 6B illustrate an embodiment of the hemostatic device in which the first and second membranes have a corrugated shape forming an "accordion structure".

In said accordion structure, the second membrane 202 comprises a plurality of flat trays 202a separated by V-shaped recesses 202b, which provide flexibility to the membrane. Similarly, the first membrane 201 comprises a plurality of flat trays 201 a facing the flat trays 202a separated by V-shaped recesses 201b facing the recesses 202b. Each pair of flat trays 201a, 202a is connected by a pillar or wall of the internal structure 204. The through holes 203 are located in the flat trays 202a and are arranged at regular interface over the circumference of the hemostatic device.

FIG. 6C illustrates the main geometric parameters of such an accordion structure.
α is the angle of the accordion structure. This angle has a direct impact on the expansion capability of the device. If α is too large, the allowable expansion will be lower. If α is too small, the area of vacuum flow will be smaller, which may lead to obstruction. In practice, α may be from about 30° to 180°.
β is the angle between each pattern of the accordion. This angle conditions the number of patterns of the accordion structure, and thus the overall flexibility of the structure.
a is the width of the trays 202a, that are "non-moving" surfaces during expansion of the hemostatic device. If a is high, expansion will be smaller. If a is low, contact hemostasis may be poorly ensured during the retraction phase. In practice, the width a may be approximately from 1 to 5 mm.
x is the accordion depth, which has an impact on the expansion capability of the device. The depth x depends directly on a, α and β.
y is the overall height of the second chamber 200, which corresponds to the height of the internal structure 204 plus the thicknesses of the first and second membranes 201, 202. In practice, y may be from 2 to 20 mm approximately.

Of course, the skilled person may design another type of accordion structure, depending on the intended expansion and retraction of the hemostatic device.

The hemostatic device may be manufactured by various processes.

Manufacturing processes include layer-by-layer processes (also known as additive manufacturing or 3D printing), molding processes, plastic injection, machining, and other plastic production methods. As regards 3D printing processes, the technology called "Carbon Digital Light Synthesis^{™}" is particularly advantageous in that it allows manufacturing biocompatible elastic parts while avoiding constraints due to molding processes. In particular, such a process allows manufacturing the base and the first and second membranes as a single part. When the hemostatic device comprises an internal structure in the first and/or second chambers, said internal structure may be integrally formed with the first and second membranes.

The material is a highly elastic polymer, with optimal mechanical characteristics such as an elongation at break greater than 250%. The invention can for example be produced with an elastomeric material comparable to thermoplastic polyurethane (TPU). Here, additive manufacturing is based on 3D models of the device, these being obtained by a preliminary phase of computer design.

This process, in which each layer is made of a biocompatible resin subjected to localized irradiation by UV light under oxygen exposure, enables the development of functional prototypes and end-use parts on a single machine.

Of course, the first and second membranes may be manufactured separately and then assembled, for example by gluing, mechanical assembly, ultrasound welding or thermal welding.

Besides, the first and second membranes may not be made by the same process.

For example, the first membrane may be formed integral with the internal structure of the second chamber (e.g. by 3D printing or injection molding), and the second membrane may be formed separately (e.g. by 3D printing or injection molding) and bonded by a medical glue to some regions of the first membrane and/or the internal structure.

Although it requires subsequent assembling, separately manufacturing the components of the hemostatic device allows optimizing the process / material for each component of the device, advantageously combining different technologies like 3D printing and injection molding.

The insertion rod and the flexible member allowing connecting the insertion rod to the base may be made by layer-by-layer method, molding processes, plastic injection, machining, and other plastic production methods. The insertion rod may be made of a substantially rigid material such as plastic or stainless steel, while the flexible member may be made of an elastomeric material.

### REFERENCES

EP 3 248 624
US 2005/0015047
US 2017/0035949
WO 2020/123525

## Claims

1. Hemostatic device (1) comprising at least a first chamber (100) configured to be fluidically connected to a fluid source and a second chamber (200) configured to be fluidically connected to a vacuum source,
wherein the hemostatic device comprises a first membrane (201) fluidically isolating the second chamber (200) from the first chamber (100) and a second membrane (202) configured to be placed so as to face, at least partially, a bleeding area of a natural body cavity, the second membrane (202) comprising a plurality of through holes (203) leading into the second chamber (200) and configured to induce a negative pressure in the natural body cavity when a negative pressure is applied in the second chamber (200) by the vacuum source, the induced negative pressure being configured so that the walls of the natural cavity are attracted to the second membrane (202).

2. Hemostatic device according to claim 1, wherein the second chamber (200) extends at least partially around the first chamber (100).

3. Hemostatic device according to claim 1, wherein the first chamber (100) extends at least partially around the second chamber (200), the first chamber (100) being enclosed between the second membrane (202) and the first membrane (201), the first membrane comprising through holes (205) in fluidic communication with the through holes (203) of the second membrane (202) by tunnels (206) passing through the first chamber (100).

4. Hemostatic device according to claim 1 or claim 2, wherein the second chamber (200) comprises an internal structure (204) mechanically connecting the first and second membranes (201, 202) to prevent collapsing of the second membrane (202) when a negative pressure is applied in the second chamber (200).

5. Hemostatic device according to claim 4, wherein the internal structure (204) comprises at least one pillar or wall extending between the first and second membranes (201, 202) so as to define, in the second chamber (200), at least two cavities (200') in fluidic communication with each other.

6. Hemostatic device according to claim 5, wherein at least one of the plurality of through holes (203) is arranged in the second membrane of each respective cavity (200').

7. Hemostatic device according to any one of claims 1 to 6, wherein the through holes (203) are arranged in the second membrane according to a regular pattern.

8. Hemostatic device according to any one of claims 1 to 7, wherein the first and second membranes (201, 202) present a corrugated shape, such that the first and second membranes are foldable in at least one direction when a compressive force is applied to the hemostatic device in said at least one direction.

9. Hemostatic device according to any one of claims 1 to 8, wherein the first and second membranes (201, 202) present a corrugated shape, such that the first and second membranes are expandable when a positive pressure is applied in the first chamber.

10. Hemostatic device according to any one of claims 1 to 9, wherein the first chamber (100) comprises an internal lattice or foam (101) selectively collapsible or expandable between a retracted configuration and an expanded configuration.

11. Hemostatic device according to any one of claims 1 to 10, further comprising a base (300) coupled to the first and second chambers (201, 202), wherein the base comprises
at least one first connecting tube (301) extending between the first chamber (201) and a first face (304) of the base, and
at least one second connecting tube (302) extending between the second chamber (202) and a second face (305) of the base.

12. Hemostatic device according to claim 11, comprising an insertion rod (400) mechanically connected to the base (300), the insertion rod comprising a first channel (401) in fluidic communication with the first connecting tube (301) and connected to the fluid source and a second channel (402) in fluidic communication with the second connecting tube (302) and connected to the vacuum source.

13. Hemostatic device according to claim 12, wherein the insertion rod (400) is connected to the base (300) by a flexible member (500).

14. Hemostatic device according to any one of claims 1 to 13, wherein the first chamber is expandable by a fluid pressure from a retracted state allowing insertion of the hemostatic device into a uterus to an expanded state fitting the internal cavity of the uterus.

15. Process for manufacturing a hemostatic device according to any one of claims 1 to 14, comprising forming the first and second membranes (201, 202) by a layer-by-layer process in which each layer is made of a biocompatible polymer.
